# EUROPEAN PATENT APPLICATION

(11) **EP 1 974 692 A1**
(43) Date of publication of application: **01.10.2008**
(21) Application number: 06757929.2
(22) Date of filing: 20.03.2006
(51) Int. Cl.: A61F 2/01

(54) **SET FOR PREVENTING A LUNG ARTERY THROMBEMBOLIA AND AN INTRAVENOUS FILTER**

(30) Priority: 28.12.2005 RU 2005141072
(71) Applicant: Andreev, Uriy Germanovich, 149680 (RU); Cherkasov, Valeriy Andreevich, Moscow (RU)
(72) Inventor: Andreev, Uriy Germanovich, 149680 (RU); Cherkasov, Valeriy Andreevich, Moscow (RU)
(74) Representative: Andrae, Steffen
(86) International application number: PCT/RU2006/000130
(87) International publication number: WO 2007/075110

(57) **Abstract**

The group of useful inventions concerns the field of medical technology, more specially to surgical instruments for catching of thromoemboli and to devices for their transport to an installation site. The a set for preventive maintenance of a thromboembolism of a pulmonary artery comprises a filter and a mounting device for its installation and extraction.

In the mounting device for the installment of the filter the pusher (3) is provided with a movement limiting stop (4), and its working block is made in the form of a capsule (5) with an axially movable therein clamping cartridge (6) and a mechanism for its movement. The working block (10) of the extractor device is made in the form of a casing (13) with an aperture and a loop extending therefrom in perpendicular direction to the axis of the extractor. In the filter, the free ends (19, 20) of the fixing and centering elements are bent outwards. The threads (23) of both elements have an identical radius of bend at the end (18). The free ends (20) of the centering element have blunted ends and are made in the form of a circular-arch segment preventing the blunted ends of the fully unfolded centering legs from touching the walls of the vena cava and being directed to the centre of the lumen of the vein. The free ends (19) of the fixing element (17) are provided with protective areas (20) located between the conical part and the bent outwards fastener part and in parallel to the axis of the vein.

## Description

The group of useful inventions concerns the field of medical technology, namely surgical instruments for catching thrombo-emboli and to devices of their delivery to an installation site, in particular, to a set (kit) for the preventive maintenance (prophylaxis) of a thromboembolism of a pulmonary artery and to its intravenous filter.

The closest in its technical essence to the proposed technical solution is a set for the preventive maintenance of a thromboembolism of the pulmonary artery, including a filter consisting of a casing, arranged in it aligning and fixing filtering cone-shaped elements with combined top ends and directed into one direction, and consisting of outwardly' extending flexible threads, regularly spaced from each other on a circle, and a device for placing the filter in a vena cava, including a bougie catheter, an adjusting canulla bound to it, a pusher movably placed therein with a working block, intended for its connection with the casing of the filter, and a device for the extraction of the filter from a vena cava, including the adjusting cannula,' a removing device with a working block movably placed therein, intended for a connection with the casing of the filter (Patent of the Russian Federation Nº 2103015, Class A 61 P 2/02, 2001).

Disadvantages of the known device are:
1. An unstable position of the filter in a vena cava lumen - immediately after its implantation, or the filter can gradually "fall" sideways.
2. The extraction of the "fallen" filter from a vena cava is inconvenient as it becomes difficult, and sometimes impossible, to throw the loop of the removing device over the hook means of the casing of the filter.
3. The filtering ability of the slanted ("fallen" sideways) filter decreases.
4. The detachment from the walls of a vena cava of a leg or the legs of the filter at its extraction is traumatic.
5. The legs (especially fixing legs) gradually extend beyond the boundaries of a vena cava into surrounding tissues, punching its wall, what can lead to phlebitis and vena cava clotting at the location of the filter.
6. The known set does not allow to exactly measure the true size of a vessel with the use of a radiological apparatus, even if it has programs for a calculation of the linear sizes of veins.

Also known is an intravenous filter, comprising a threadlike casing, at one of its ends a first filtering element (centering), consisting of flexible threads (wires) diverging in the form of a cone and located on equal distances on a circle with respect to each other, and at the other end of the casing - a second filtering element (locking), from flexible threads diverging around the casing, the threads of which are located on equal distances on a circle with respect to each other and staggered under an angle in relation to the threads of the first filtering element, the free ends of these threads being bent inwards, whereas the free ends of a thread of the first filtering element are bent outwards and form a fastening tip (application EP 0 270 432 A1, class A 61 P 2/02, 1988).

However the known device has a number of essential disadvantages:
1. The possibility of slanting of the centering element, which leaves the catheter first, during installation, what leads to an impairment of the filtering ability of the filter.
2. The possibility of a bending of the vein at the exit of the centering element from the catheter what leads to a disturbance of the laminar blood stream and to the formation of thrombi.

The closest with respect to the technical nature of the proposed technical solution is the intravenous filter consisting of a casing, arranged therein centering and locking filtering, cone-shaped elements, with combined with each other ends and diverging to one side flexible threads, which are located in regular intervals to each other on a circle (patent of the Russian Federation Nº 2103015, class A 61 P 2/02, 2001).

A disadvantage of the known intravenous filter is the traumatic effect of the removal of the filter when the sharp hooks of the fixing legs are detached from the wall of a vena cava.

The problems, solved by the proposed group of useful inventions, are the creation of a design for a set for preventive maintenance of a thromboembolism of a pulmonary artery and its intravenous filter, providing:
- reliable fixing of the distal part of the delivering device to the filter, allowing both to move out of, as well as to remove in the lumen of a delivering cannula a small size self-aligning filter when it is necessary to relocate it in different sections of a vena cava up to the detachment of the filter from the delivering device,
- reliable fixing of the filter to the extraction device, allowing a withdrawal of the filter, without obstacles, from a vena cava by a withdrawal cannula,
- conservation of the maximum filtering properties of the filter due to its reliable self-centering.

### Disclosure of the invention

The technical result of the proposed invention enables the creation of a set for the preventive maintenance (prophylaxis) of a thromboembolism of the pulmonary artery, including the filter consisting of the casing, established in it aligning and fixing filtering cone-shaped elements, with their combined ends and directed to one side and consisting of diverging flexible threads in regular intervals located on a circle between each other, a device for the filter installment in a vena cava, including a bougie catheter, the adjusting cannula bound to it, movably placed in it a pusher with a working block intended for connecting with the casing of the filter, and a device for the extraction (withdrawal) of the filter from a vena cava, including the extracting cannula, a movably placed therein withdrawing device with a working block intended for connecting with the casing of the filter, in which, according to the inventions, the pusher of the device for the filter installment in a vena cava is supplied with a limiting stop of its movement, and its working block is made in the form of a capsule with an axially movable clamping cartridge provided therein, the working block of the withdrawing device being made in the form of a casing with an aperture for the loop of the withdrawing device extending therefrom perpendicularly to its axis.

Providing the pusher of the device for the filter installment in a vena cava with a limiting stop of its movement makes it possible to push it out for the necessary quantity of times from the adjusting cannula in a vena cava lumen (to straighten it) and to remove it in the adjusting cannula from a vena cava (bring the filter in its compact condition) for the purpose of a more exact choice of the place of its implantation and the most adequate unfolding of the filter in a vein when it is strongly deformed.

Providing the working block in the form of a capsule with established in it with a possibility of its axial movement of a clamping cartridge allows it to fix reliably the filter to the delivering device.

The proposed design of the working block of the working device gives the chance to direct a metal loop strictly perpendicularly to the lumen of a vena cava for a reliable capture of a back part of the filter, and also, thanks to the presence in the block of a groove, strictly coaxially to catch with the loop the back part of the filter.

It provides an easy sliding of an extracting cannula onto the filter without the filter moving in a longitudinal direction, and also a reliable and non-traumatic detachment developing thus to the centre of a vena cava of fixing legs from vena cava walls as for this moment for the account of centering legs remains in the center a extracting cannula in a vena cava lumen, and centering legs start to develop in a fascicle already after the compression of the fixing legs. Thus it is possible to freely extract the filter from a vena cava in an extracting cannula.

The proposed invention is characterized also in that the set is supplied with a mechanism for the measurement of the diameter of the vein, located on an adjusting cannula, the mechanism being made in the form of two separated in a distance from each other radiopaque labels.

This allows it to make an exact measurement of the absolute size of a lumen of a vena cava with the use of any interventional apparatus.

The proposed invention is characterized also in that the mechanism of movement of the clamping cartridge is supplied witn a safety means intended for the prevention of a spontaneous detachment of the filter from the delivering device.

The provision of the mechanism of movement of the clamping cartridge in the form of a thread, which is connected to a rest, simplifies the design of the device for the filter installment in a vena cava.

The provision of a safety means in the form of the cylinder with the lateral cut, established on a thread behind the rest fixed to it allows it to limit at the same time the travel of the pusher and to keep it in the necessary position.

The invention is **characterized in that** clamping the cartridge can be made in a kind of clamping grip.

Such performance of the cartridge is necessary for the proposed construction of the filter and allows to keep reliably the filter and quickly to disconnect the filter from a pusher.

The technical result of the proposed invention also provides an intravenous filter consisting of a casing, arranged in it aligning and fixing filtering, cone-shaped elements with their combined ends and directed to one side and consisting of diverging flexible threads located on a circle in regular intervals between each other in which, according to the invention, the free ends of the fixing and aligning elements are bent outwards such that the threads of both elements have an identical radius of bend at the top end, and the free ends of the aligning elements have blunted ends and are made in the form of an circular-arc segment allowing the blunted ends of the fully unfolded centering legs not to touch the walls of a vena cava and to be directed to the centre of the lumen of the vein, and the free ends of the fixing elements being supplied by a protective area located between the conical part and the bent outwards hook part and in parallel to the axis of the vein.

The proposed construction of the filter avoids reliably a trauma and it is safe to attached the filter to a vein wall due to the form of the fixing legs and the presence at them of protective areas.

The proposed filter always takes a coaxial-central position in a vena cava lumen, i.e. without inclinations, and keeps the maximum filtering ability of the filter because of its coaxial-central position, and also maximally narrow gaps in the cone-shaped part of the filter in the zone of the maximum (laminar) stream of blood.

The small height of the size of the filter allows it to implant it on the site of the vena cava located directly below the level of the confluence of the renal veins of limited extent,what is very important at high diffusion «fluttering» thrombus apexes in the lumen of the lower vena cava.

Supplying the intravenous filter with radiopaque labels which are established on the blunted ends of the centering legs allows an enlargement of the contrast of the filter.

The combined use of the proposed invention allows:
- to lower considerably the risk of a thrombogenesis on the filter since there are no extra elements in the zone of the laminar stream of blood (hooks, loops, a carving etc.).
- to simplify the procedure of extracting the filter from a vena cava, not causing damage to the vascular wall and the filter;
- to fix reliably, as well as to disconnect reliably the filter from the delivering device due to the peculiarities of its design;
- to use an implanting cannula with small external diameter;

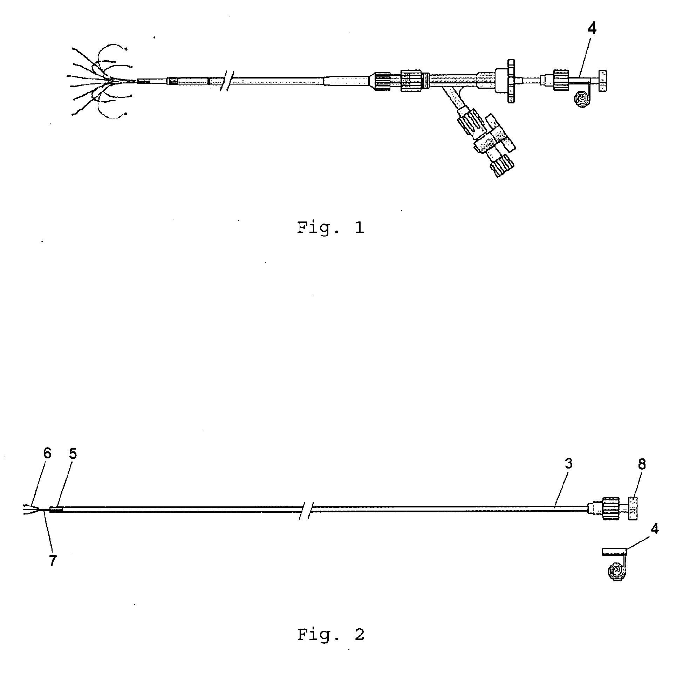

The essence of the proposed set for the preventive maintenance of a thromboembolism of a pulmonary artery and the intravenous filter is explained by the below-mentioned description of designs and drawings, where
On Fig.1 - the scheme of a set for preventive maintenance of a thromboembolism of a pulmonary artery is shown;
On Fig.2 - the scheme of the device for the filter installment in a vena cava is shown;

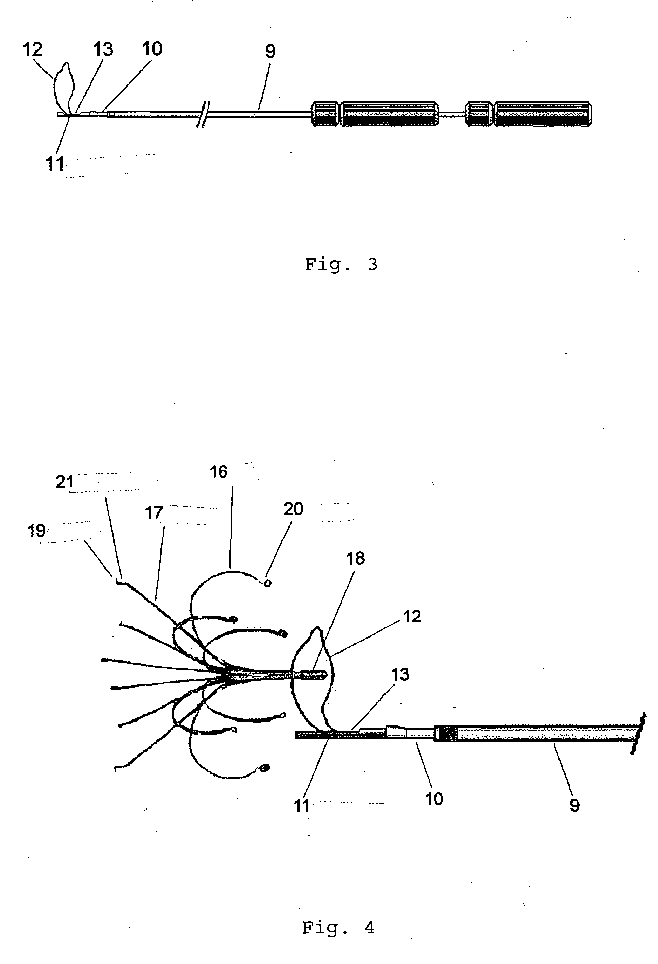

On Fig.3 - the scheme of the device for extraction of the filter from a vena cava is shown;
On Fig.4 - the design of the intravenous filter with the device for extraction is shown.

### The best embodiment

The set for preventive maintenance of a thromboembolism of a pulmonary artery includes the intravenous filter, the device for the filter installment in a vena cava, and the device for extraction of the filter from a vena cava.

The set can be supplied with a mechanism for the measurement of the diameter of the vein, located on an adjusting cannula.

The device for the filter installment in a vena cava consists of a boogie catheter 1, the adjusting cannula 2 bound to it, the pusher 3 placed in it with a working block intended for its connection with the filter, a limiting stop for the movement of the pusher 4.

The working block is made in the form of a capsule 5, with a clamping cartridge 6 provided therein and a mechanism of its longitudinal movement connected to it.

The mechanism of movement of the clamping cartridge is made in the form of a thread 7 of which one end is bound to the clamping cartridge 6, and the other is fixed to the rest 8.

One the embodiments of the clamping cartridge 6 can be a gripping clamp.

The limiting stop of the movement of the pusher 4 can be made in the form of a casing, for example, of cylindrical form, with a lateral cut which is established on the mechanism of movement of the clamping cartridge, for example, on a thread 7.

The device for the extraction of the filter from a vena cava consists of the extracting cannula 9, a movably placed therein extraction device 10 with a working block intended for a connection with the filter.

The working block of the extracting device is made in the form of the casing with an aperture 11 and a loop 12 leaving it perpendicularly to the axis of the extracting device.

The casing of the working block of the extracting device is made in the form of a groove 13 allowing to strictly coaxially connecting the drawing loop 12 with the back part of the filter (end of elements).

The mechanism of measurement of the diameter of a vein is made in the form of two separated in a distance from each other radiopaque labels 14 and 15.

The intravenous filter includes established in its aligning 16 and fixing 17 filtering, cone-shaped elements, the ends of which 18 are combined, and their diverging flexible threads with their free ends being directed to one side and circumferentially located in regular intervals between each other.

Thus the free ends of the fixing 19 and centering elements 20 are bent outwards such that the threads of both elements have an identical radius of bend at the top end 18.

The free ends of the aligning 20 elements have blunted ends and are made in the form of a circular-arc segment allowing the blunted ends of the fully unfolded centering legs not to touch of walls of a vena cava and to be directed to the centre of the lumen of the vein.

The free ends of the fixing elements 19 are supplied with a protective area (plateau) 21 located between the conical part and the bent outwards hook part and a in parallel to the axis of the vein.

The filter can be supplied with radiopaque labels established on the blunted ends the of centering legs 20.

### The proposed group of useful inventions works as follows:

At the time of the radiological intervention after a vascular puncture by technique Seldinger under x-ray television control a transcutaneous catheterization of an adjusting cannula 2 through subclavial (more preferably the left), right internal bulbar or one of femoral veins is conducted.

The distal end of the cannula enters into a vena cava lumen up to the approximate level of implantation of the filter.

Adjusting cannula 2 with radiopaque labels 14 and 15, made on its distal end, establish in the lower vena cava, for example, on 5 mm more low to the beginning of renal veins, being based on data inferior veno-cavagraphy, and also a selective retrograde catheterization of renal veins and fluoroscopy. The bougie 1 is delete with a wire.

The filter in the capsule 5 of the device for the filter installment in a vena cava is connected to the clamping cartridge 6 and the mechanism of its longitudinal movement is bound to it.

The device for the installment of the filter with the filter fixed on its end under the control x-ray television is delivered to the distal end of an adjusting cannula 2.

In this position the adjusting cannula 2 is kept motionless, and the filter with the clamping cartridge 6 is slowly advanced.

Thus the free ends of the centering element 16 of the filter, being released, open under the ostia of the renal veins, aligning the device for the installment of the filter as well as the filter in the lumen of the lower vena cava.

Before fully releasing the free ends of the fixing element 17 more exact correction of the level of the installment of the filter in the infra renal part of the lower vena cava is possible.

Thus radiopaque labels, for example, platinum on the ends of an aligning element should be located below the level of the confluence of the renal veins.

Then the device for the filter installment is fixed in a vena cava on a place, and an adjusting cannula 2 tighten on itself before full disclosing of the filter and a full exit of a capsule 5 of a lumen of an adjusting cannula 2.

Under the fluoroscopic control (it is better to produce a control x-ray film) one can become convinced that the filter is located along an axis of the lower vena cava at the required level.

The limiting stop 4 of the movement of the pusher is taken out, and the filter is disconnected from the capsule 5 of the device for the filter installment in a vena cava, by a fixed to a rest 8 thread 7, and the device for the filter installment tightens itself. Thus clamping the cartridge, revealing, is disconnected from the filter.

The proposed designs of the filter and the device for the filter installment in a vena cava allow to change the position of the filter in the course of its installment in the lower vena cava provided that the filter is not disconnected from the capsule 5.

For correcting the position of the filter the device for the filter installment in a vena cava is kept motionless, and an adjusting cannula 2 is displaced forward against the stop while threads centering 16 and fixing 17 elements completely will not develop, and will not interfere with the movement of an adjusting cannula 2 with the filter on the bottom vena cava.

Thereby, favorable conditions for a more exact choice of the level of implantation of the filter are provided.

To change of the position of an adjusting cannula 2 in the lower vena cava is possible also after excision from it the device with the filter completely. This reception is necessary in casings which are a vena cava when moving of an adjusting cannula on a vein should be carried out on a conductor.

After a filter detachment delivering device delete. An adjusting cannula 2 also delete or leave for carrying out of a control angiography (it is desirable in two projections).

After excision of an adjusting cannula 2 spend a hemostasis manual pressing of a place of a puncture.

### Extraction of the intravenous filter make as follows:

Cannula introduction through subclavial (it is more preferable at the left), right internal bulbar, and also femoral veins make in the same way as it is described earlier.

However, thus apply extracting cannula from a set to extraction of the filter from a vena cava. The distal end of a cannula bring to the filter.

Preliminary a loop 12 of the withdrawing device, leaving the casing of the withdrawing device involve in a trench of the casing 13. Into a lumen of a extracting cannula 9 enter withdrawn device 10 with the working block. Under the control fluoroscopy a loop 12 of the withdrawing device open over the end of the filter and in the opened kind get for it. Then tighten on itself the working block of the withdrawing device and a loop close, grasping and densely drawing to the block of the withdrawing device the end of the filter 18. Extracting cannula 9 is pulled over the filter before its full retraction into a cannula lumen. Then the cannula 9 is ectracted with the withdrawing device 10 and the filter is deleted.

The proposed group of useful inventions has been clinically tested in three large clinics of Moscow (the Main military clinical hospital of Burdenko, 3 Central clinical hospital of Vishnevsky, chair of surgical illnesses RGMU) and the obtained results have confirmed the reliability, simplicity of the installment of the filter, an accurate centering along a vein axis, an considerable decrease of the risk of a thrombogenesis on the filter, conservation of the maximum filtering ability.

## Claims

1. Set for the preventive maintenance of a thromboembolism of the pulmonary artery, including
a filter consisting of a casing (22), arranged therein cone-shaped centering (16) and fixing (17) filtering elements with combined ends (18) and directed to one side and made from diverging flexible threads circumferentially located in regular intervals with respect to each other,
a device for the installment of the filter in a vena cava, including a bougie catheter (1), an installment cannula (2) connected therewith, a movably arranged therein pusher (3) with a mounting block for engaging the casing (22) of the filter, and
a device for the extraction of the filter from a vena cava, including an installment cannula (9), a movably arranged therein extractor device (10) with a working block for engaging the casing of the filter,
**characterized in that** the pusher (3) of the device for the installment of the filter in a vena cava is provided with a movement limiting stop (4), its working block being made in the form of a capsule (5) with an arranged therein clamping cartridge (6) and a mechanism for its axial movement, whereas the working block (10) of the extractor device is made in the form of a casing (13) with an aperture (11) and an extractor loop (12) extending therefrom in a perpendicular direction.

2. Set according to claim 1, **characterized in that** it is supplied with a mechanism for measuring the diameter of the vein, located on the installment cannula.

3. Set according to claim 1 and 2, **characterized in that** the mechanism for measuring the diameter of the vein is provided in the form of two arranged in a distance from each other radiopaque labels (14, 15).

4. Set according to claim 1, **characterized in that** the mechanism for the axial movement of the clamping cartridge is in the form of a thread (7) connected to a rest (8).

5. Set for the preventive maintenance of a thromboembolism of a pulmonary artery according to claim 1, **characterized in that** the clamping cartridge (6) is in the form of a plier clamp.

6. Set according to claim 1, **characterized in that** the movement limiting stop of the pusher (4) is in the form of a casing, for example of cylindrical form, with a lateral cut, provided on the mechanism for the movement of the clamping cartridge.

7. Filter including a casing (22), consisting of cone-shaped centering (16) and fixing (17) filtering elements with combined ends (18) and directed to one side and formed of diverging flexible threads (23) circumferentially located in regular intervals with respect to each other,
**characterized in that** the free ends (19,20) of the fixing and centering elements are bent outwards, the threads (23) of both elements having an identical radius of bend at the end (18), the free ends (20) of the centering element having blunted ends and being made in the form of a circular arc segment preventing the blunted ends upon full unfolding of the centering legs from touching the walls of the vena cava and being directed to the centre of the lumen of the vein, whereas the free ends (19) of the fixing element (17) are provided with a protective area (21) located between the conical part and the bent outwards fastener part and arranged in parallel to the axis of the vein.

8. Filter according to claim 7, **characterized in that** its fixing element is provided with radiopaque labels (24).
